# FASCICULE DE BREVET EUROPEEN

(11) **EP 1 068 858 B1**
(45) Date de publication et mention de la délivrance du brevet: **13.04.2005**
(21) Numéro de dépôt: 00401744.8
(22) Date de dépôt: 19.06.2000
(51) Int. Cl.: A61K 7/06

(54) **Utilisation de l'acide 4,6-dimethoxy-indole 2-carboxylique ou de ses dérivés pour stimuler ou induire la pousse des cheveux et/ou stopper leur chutte**
Verwendung der 4,6-Dimethoxyindol-2-carbonsäure zur Induzierung und/oder Stimulation des Haarwachstums und/oder zur Verhinderung des Haarausfalls
Use of 4,6-dimethoxyindole-2-carboxylic acid for stimulating or inducing hair growth and/or arresting hair loss

(30) Priorité: 16.07.1999 FR 9909268
(43) Date de publication de la demande: 17.01.2001
(73) Titulaire: L'OREAL, 75008 Paris (FR)
(72) Inventeur: Dalko, Maria, 91190 Gif-sur-Yvette (FR); Galey, Jean-Baptiste, 93600 Aulnay-sous-Bois (FR); Bernard, Bruno, 92200 Neuilly-sur-Seine (FR)
(74) Mandataire: Galup, Cédric Olivier Nicolas

(56) Documents cités:
- EP-A- 0 470 490
- WO-A-99/07351
- WO-A-99/12905
- D. A. HOLT ET AL: "Benzophenone- and indolecarboxylic acids: potent type-2 specific inhibitors of human steroid 5alpha-reductase" JOURNAL OF MEDICINAL CHEMISTRY, vol. 38, no. 1, 1995, pages 13-15, XP002068506 WASHINGTON US

## Description

L'invention concerne l'utilisation cosmétique dans une composition ou pour la préparation d'une composition pharmaceutique d'une quantité efficace de l'acide 4,6-diméthoxy-indole 2-carboxylique ou de ses dérivés, le composé ou la composition étant destinés à induire et/ou stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Cependant différentes causes peuvent entraîner une perte importante, temporaire ou définitive, des cheveux. L'alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

Le terme alopécie recouvre toute une famille d'atteintes du follicule pileux ayant pour conséquence finale la perte définitive partielle ou générale des cheveux. Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

On recherche depuis de nombreuses années, dans l'industrie cosmétique ou pharmaceutique, des substances permettant de supprimer ou de réduire l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Des composés particuliers de la famille des indole-carboxyliques, comme ceux décrits dans la demande de brevet WO-A-9912905, ont également été proposés pour leur capacité à induire et/ou stimuler la croissance des cheveux et/ou freiner leur chute.
Ces composés présentent une activité inhibitrice marquée des 5α-réductases de type I et II, ce qui en fait suivant la théorie qui veut que ces protéines soient impliquées dans la chute des cheveux, d'excellents candidats comme principe actif pour induire et/ou stimuler la croissance des cheveux et/ou freiner leur chute.
Cependant, les 5α-réductases ne sont pas exclusivement présentent au niveau du follicule pileux et l'on comprend des lors tout l'intérêt de disposer de composés capables d'induire et/ou de stimuler la croissance des cheveux et/ou de freiner leur chute, mais ne présentant pas d'activité sur les 5α-réductases de type I et II.

La demanderesse a de manière surprenante et inattendue découvert que l'acide 4,6-diméthoxy-indole 2-carboxylique ou ses dérivés présentent la propriété d'induire et/ou de stimuler la croissance des cheveux et/ou de freiner leur chute, mais ne présentant pas d'activité sur les 5α-réductases de type I et de type II.

Ainsi, la présente invention a pour objet l'utilisation cosmétique dans une composition, d'une quantité efficace d'au moins un composé répondant à la formule générale (I) : 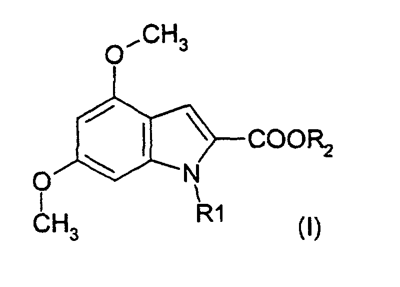 dans laquelle
R₁ et R₂, identiques ou différents, représentent :
un atome d'hydrogène,
ou un radical alkyle en C₁-C₆, éventuellement substitué par un radical -OH, -NHR₃, -SH, -COOH ou -COOR₃, dans lesquels R₃ représente un radical alkyle en C₁-C₄, linéaire ou ramifié,
ou un radical aralkyle en C₇ - C₁₂,
ou un radical -CHR₄R₅, dans lequel R₄ et R₅ identiques ou différents représentent un atome d'hydrogène ou un radical phényle éventuellement substitué ou encore un hétérocycle ayant 5 ou 6 chaînons ;
ses formes acylées ou encore ses sels physiologiquement acceptables pris seuls ou en mélange en toutes proportions ;
ledit composé et/ou lesdites compositions étant destinés à induire et/ou stimuler la croissance des cheveux et/ou freiner leur chute.

La présente invention a également pour objet l'utilisation d'un quantité efficace d'au moins un composé répondant à la formule I, telle que définie ci-dessus, pour la préparation d'une composition pharmaceutique destinée à traiter la chute des cheveux.

Ces composés présentent des activités remarquables qui justifient leur utilisation comme principe actif pour induire et/ou stimuler la croissance des cheveux et/ou freiner leur chute.

A la connaissance de la demanderesse il n'a jamais été proposé dans l'art antérieur l'utilisation de tels composés pour lutter contre la chute des cheveux.

Par hétérocycle, on entend de préférence selon l'invention un cycle incluant éventuellement un ou plusieurs atomes d'azote et/ou d'oxygène et particulièrement la pyridine, l'imidazole, le tétrahydrofuranne ou le furanne. Un hétérocycle particulièrement préféré selon l'invention est la pyridine.

Par radical alkyle en C₁-C₄ on entend selon l'invention les radicaux acycliques provenant de l'enlèvement d'un atome d'hydrogène dans la molécule d'un hydrocarbure, linéaires ou ramifiés ayant de 1 à 4 atomes de carbone et en particulier les radicaux méthyle, éthyle, propyle, isopropyle, butyle, isobutyle ou tertiobutyle.

Par radical aralkyle en C₇ - C₁₂, on entend de préférence selon l'invention les radicaux alkyles-aryles ayant de 7 à 12 atomes de carbone, définition dans laquelle le terme aryle s'entend comme un cycle aromatique ayant 5 ou 6 atomes de carbone ou un hétérocycle aromatique ayant 5 ou 6 atomes. Préférentiellement selon l'invention, le radical aralkyle est en C₇-C₁₀. Un radical aralkyle particulièrement préféré selon l'invention est le radical benzyle.

Par un radical phényle éventuellement substitué, on entend de préférence selon l'invention le radical phényle éventuellement substitué par un groupement cyano (-CN), un groupement trifluorométhyle (-CF₃), un radical méthoxy (-O-CH₃) ou un atome d'halogène. L'atome d'halogène peut être choisi parmi le chlore, le brome, le fluor, l'iode. Un radical phényle substitué particulièrement préféré selon l'invention est le radical phényle substitué par un groupement trifluorométhyle (-CF₃).

Selon une forme de réalisation préférée de l'invention R₁ représente un atome d'hydrogène ou un radical méthyle ou bien éthyle.

Selon une autre forme de réalisation préférée de l'invention R₂ représente un atome d'hydrogène ou un radical méthyle.

Selon une forme de réalisation très préférentielle de l'invention, R₁ et R₂ sont un atome d'hydrogène.

On peut citer comme composés de formule (I) :
l'acide 4,6-diméthoxy-indole 2-carboxylique
l'acide 4,6-diméthoxy-indole 2-carboxylate de méthyle
l'acide N-méthyl-4,6-diméthoxy-indole 2-carboxylique
l'acide N-méthyl-4,6-diméthoxy-indole 2-carboxylate de méthyle
l'acide N-éthyl-4,6-diméthoxy-indole 2-carboxylique.

Parmi ces composés, on préfère tout particulièrement l'acide 4,6-diméthoxy-indole 2-carboxylique

Selon l'invention, les composés peuvent être utilisés seuls ou en mélange.

La quantité efficace de composé à utiliser correspond bien entendu à la quantité nécessaire pour obtenir le résultat désiré. L'homme du métier est donc en mesure d'évaluer cette quantité efficace qui dépend de la nature du composé utilisé et de la personne ainsi traitée.
Pour donner un ordre de grandeur, dans les compositions selon l'invention le composé est présent à une concentration comprise entre 0,3% et 30% en poids par rapport au poids total de la composition et de préférence comprise entre 0,5% et 20%.

Selon l'invention, les composés de formule (I) peuvent être utilisés dans des compositions à usage cosmétique ou pharmaceutique. Préférentiellement selon l'invention, les composés de formule (I) sont utilisés dans des compositions à usage cosmétique.

Le milieu physiologiquement acceptable dans lequel l'actif est utilisé selon l'invention est anhydre ou aqueux. On entend par milieu anhydre, un milieu solvant contenant moins de 1% d'eau. Ce milieu est constitué d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 6 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable puisse contenir d'autres adjuvants habituellement utilisés dans le domaine cosmétique ou pharmaceutique, tels que des agents tensioactifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.

Il est possible aussi d'utiliser en association avec l'actif des composés améliorant encore l'activité sur la repousse et/ou sur le freinage de la chute des cheveux, et ayant déjà été décrits pour cette activité.

Parmi ces derniers composés, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3-oxyde ou "Minoxidil" décrit dans les brevets US 4 139 619 et US 4 596 812 ;
- les agents favorisant la repousse des cheveux comme ceux décrits par la demanderesse dans la demande de brevet européen publiée sous le numéro 0648488 ;
- les agents antibactériens tels que les macrolides, les pyranosides et les tétracyclines, et notamment l'Erythromycine ;
- les agents antagonistes de calcium, comme la Cinnarizine, le Diltiazem, la Nimodipine et la Nifedipine ;
- des hormones, telles que l'estriol ou des analogues, ou la thyroxine-et ses sels ;
- des agents anti-inflammatoires stéroïdiens, tels que les corticostéroïdes (par exemple : l'hydrocortisone) ;
- des agents antiandrogènes, tels que l'oxendolone, la spironolactone, le diéthylstilbestrol et la flutamide ;
- des inhibiteurs stéroïdiens ou non stéroïdiens des 5-α-réductases tels que le finastéride
- des agonistes potassiques tels que la cromakalim et le nicorandil.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un radical alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoïdes, les acides eicosatétraénoïque et eicosatriénoïque ou leurs esters et amides, la vitamine D et ses dérivés, des extraits d'origine végétale ou bactérienne.

On peut également envisager que la composition comprenant au moins un composé tel que défini précédemment soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition cosmétique selon l'invention peut être appliquée sur les zones alopéciques du cuir chevelu et des cheveux d'un individu, et est éventuellement laissée en contact plusieurs heures et est éventuellement à rincer. On peut, par exemple, appliquer la composition contenant une quantité efficace d'au moins un composé tel que défini précédemment, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu, caractérisé par le fait qu'il consiste à appliquer sur les cheveux et/ou le cuir chevelu, une composition cosmétique comprenant au moins composé tel que défini précédemment, à laisser celle-ci en contact avec les cheveux et/ou le cuir chevelu, et éventuellement à rincer.

Le procédé de traitement présente les caractéristiques d'un procédé cosmétique dans la mesure où il permet d'améliorer l'esthétique des cheveux en leur donnant une plus grande vigueur et un aspect amélioré.

On va maintenant donner à titre d'illustration des exemples qui ne sauraient limiter en aucune façon la portée de l'invention.

Exemple : évaluations comparées de l'effet inhibiteur de l'acide 4,6-diméthoxy-indole-2-carboxylique et du 5-méthoxy-6-benzyloxy-indole-2-carboxylique sur les 5α-réductases de types I et II :

Les séquences codantes (Acides désoxyribonucléiques complémentaires : cDNA) de la 5α-réductase 1 et de la 5α-réductase 2 ont été clonées dans le vecteur d'expression eucaryote pSG5 (Stratagene). Les enzymes ont été surexprimées après transfection transitoire de cellules COS7 (ATCC, CRL1651).

Les cDNAs de la 5α-réductase 1 et de la 5α-réductase 2 ont été obtenus par transcription inverse et réaction de polymérisation en chaîne (Polymerase chain reaction : PCR) à l'aide d'amorces spécifiques à partir d'ARN total de testicules humains (vendu par la société Clontech).
Les amorces utilisées pour obtenir le cDNA de la 5α-réductase 1 sont : Brin + : 5' CCCAGCCCTGGCGATGGCAAC 3', Brin - : 5' GGATATTCAACCTCCATTTCAG 3'. Les amorces utilisées pour obtenir le cDNA de la 5α-réductase 2 sont :

Brin + : 5' GCGATGCAGGTTCAGTG 3',

Brin - : 5' ATTGTGGGAGCTCTGCT 3'.

Par des techniques classiques de génie génétique, le cDNA de la 5α-réductase 1 obtenu a été inséré au site BamHI de pSG5 et celui de la 5α-réductase 2 au site EcoRI (voir Magnatis et col., Molecular Cloning, Cold Spring Harber, 1989)

Les clones positifs (recombinants) ont été identifiés par la technique d'hybridation avec une sonde froide (Plex luminescent kits, Millipore) et cartographiés par digestions enzymatiques et séquençage partiel. Après transfection transitoire les cellules COS7 sont lysées dans un tampon Tris-HCl 10 mM, pH=7 / 150 mM KCI / 1 mM EDTA par 3 cycles de congélation/décongélation. L'homogénat est centrifugé à 100 000 g pendant 1 heure. Les culots contenant la 5α-réductase 1 ou la 5α-réductase 2 sont repris dans un tampon phosphate 40 mM, pH 6.5 ou citrate 40 mM, pH 5.5 respectivement pour la 5α-réductase 1 ou 5α-réductase 2.
5 µg de protéines ainsi obtenues sont incubés dans un puits d'une plaque 96 puits (NUNC) en présence de 1 nM de ¹⁴C-Testostérone (Amersham) et de 5 mM de Nicotinamide Adenosine Dinucleotide Phosphate, forme réduite (NADPH) (Sigma) dans le tampon correspondant (tampon phosphate 40 mM, pH 6.5 ou citrate 40 mM, pH 5.5 respectivement pour la 5α-réductase 1 ou 5α-réductase 2) pendant 50 minutes à 37°C après addition des produits à tester.
Les produits à tester sont ajoutés aux concentrations de 10⁻⁴ M à 10⁻¹⁰M, dilués dans du tampon phosphate 40 mM, pH 6.5 ou citrate 40 mM, pH 5.5 respectivement pour la 5α-réductase 1 ou 5α-réductase 2.
Les mélanges réactionnels sont ensuite déposés directement sur un plaque de silice (HPTLC, 60F 254, Merck) et soumis à une chromatographie (solvant = 10% diéthyléther, 90% dichlorométhane). Ils sont ensuite analysés par autoradiographie digitale (Digital Autoradiography, Berthold).
On mesure l'inhibition de l'activité des isoenzymes en calculant le pourcentage de dihydrotestostérone formée à partir de ¹⁴C-testostérone par rapport à un témoin non traité.
Les résultats sont présentés dans le tableau suivant :

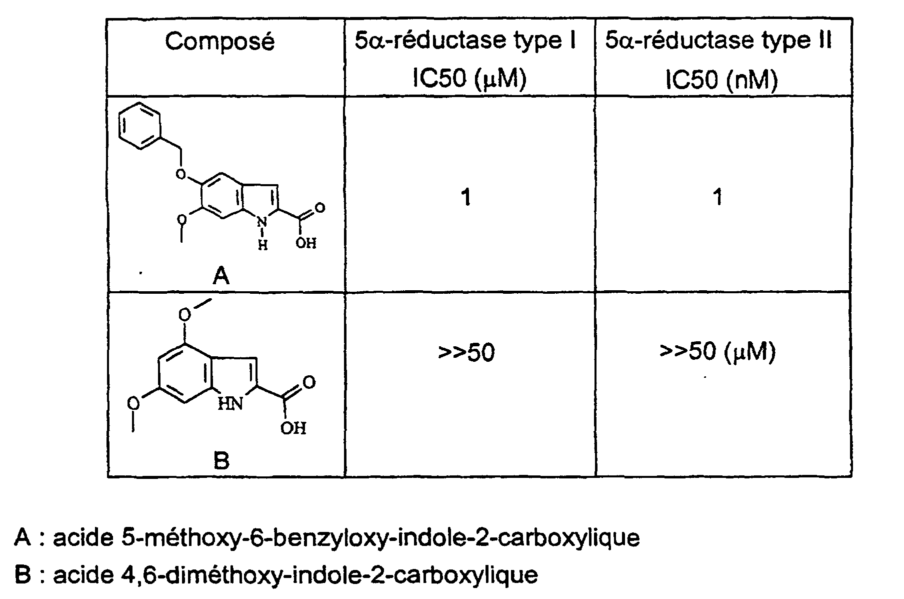

L' acide 4,6-diméthoxy-indole-2-carboxylique ne présente pas d'effet inhibiteur sur les 5α-réductases.

Exemples de compositions selon l'invention.
Ces compositions sont obtenues par les techniques habituelles couramment utilisées en cosmétique ou en pharmacie.

### Lotion:

- acide 4,6-diméthoxy-indole-2-carboxylique 5,00 g
- Propylène glycol 10,00 g
- Alcool isopropylique qsp 100,00 g
On applique 1 ml de cette lotion sur le cuir chevelu, à la fréquence de une à deux fois par jour.

### Lotion:

- acide 4,6-diméthoxy-indole-2-carboxylique 1,00 g
- Propylène glycol 30,00 g
- Alcool éthylique 40,00 g
- Eau qsp 100,00 g

On applique cette lotion sur le cuir chevelu, une à deux fois par jour, à raison d'1 ml par application.

### Lotion épaissie :

- acide 4,6-diméthoxy-indole-2-carboxylique 1,00 g
- Kawaïne 2,00 g
- Klucel G®* 3,50 g
- Alcool éthylique qsp 100,00 g

* : Hydroxypropylcellulose vendue par la société Hercules

On applique cette lotion épaissie sur le cuir chevelu, une à deux fois par jour, à raison d'1 ml par application.

### lotion :

- acide 4,6-diméthoxy-indole-2-carboxylique 2,00 g
- Dowanol PM®** 20,00 g
- Klucel G®* 3,00 g
- Alcool éthylique 40,00 g
- Eau qsp 100,00 g

** : Monométhyléther de propylèneglycol vendu par la société Dow Chemical

On applique cette lotion épaissie sur le cuir chevelu, une à deux fois par jour, à raison d'1 ml par application.

### Lotion:

- acide 4,6-diméthoxy-indole-2-carboxylique 1,00 g
- Propylène glycol 30,00 g
- Alcool éthylique 40,00 g
- Eau qsp 100,00 g

On applique cette lotion sur le cuir chevelu, une à deux fois par jour, à raison d'1 ml par application.

## Revendications

1. Utilisation cosmétique dans une composition, d'une quantité efficace d'au moins un composé répondant à la formule générale (I) : 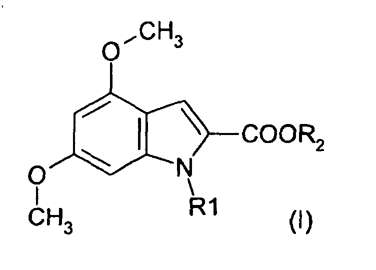 dans laquelle
R₁ et R₂, identiques ou différents, représentent :
un atome d'hydrogène,
ou un radical alkyle en C₁-C₆, éventuellement substitué par un radical -OH, -NHR₃, -SH, -COOH ou -COOR₃, dans lesquels R₃ représente un radical alkyle en C₁-C₄, linéaire ou ramifié,
ou un radical aralkyle en C₇ - C₁₂,
ou un radical -CHR₄R₅, dans lequel R₄ et R₅ identiques ou différents représentent un atome d'hydrogène ou un radical phényle éventuellement substitué ou encore un hétérocycle ayant 5 ou 6 chaînons ;
ses formes acylées ou encore ses sels physiologiquement acceptables pris seuls ou en mélange en toutes proportions ;
pour induire et/ou stimuler la croissance des cheveux et/ou freiner leur chute.

2. Utilisation d'une quantité efficace d'au moins un composé répondant à la formule générale (I) telle que définie dans la revendication 1 pour la préparation d'une composition pharmaceutique pour traiter la chute des cheveux.

3. Utilisation selon l'une des revendications 1 ou 2, **caractérisée par le fait que** R₁ est un atome d'hydrogène ou un radical méthyle ou bien éthyle.

4. Utilisation selon la revendication précédente, **caractérisée par le fait que** R₁ est un atome d'hydrogène.

5. Utilisation selon l'une quelconque des revendications précédentes, **caractérisée par le fait que** R₂ est un atome d'hydrogène ou un radical méthyle.

6. Utilisation selon la revendication précédente, **caractérisée par le fait que** R₂ est un atome d'hydrogène.

7. Utilisation selon l'une quelconque des revendications précédentes, **caractérisée par le fait que** le composé est choisi parmi
l'acide 4,6-diméthoxy-indole 2-carboxylique
l'acide 4,6-diméthoxy-indole 2-carboxylate de méthyle
l'acide N-méthyl-4,6-diméthoxy-indole 2-carboxylique
l'acide N-méthyl-4,6-diméthoxy-indole 2-carboxylate de méthyle
l'acide N-éthyl-4,6-diméthoxy-indole 2-carboxylique.

8. Utilisation selon la revendication précédente, **caractérisée par le fait que** le composé est l'acide 4,6-diméthoxy-indole 2-carboxylique.

9. Utilisation selon l'une quelconque des revendications précédentes, **caractérisée par le fait que** le composé présent est utilisé à une concentration comprise entre 0,3% et 30%, en poids par rapport au poids total de la composition.

10. Utilisation selon l'une quelconque des revendications précédentes, **caractérisée par le fait que** le composé présent est utilisé à une concentration comprise entre 0,5% et 20%, en poids par rapport au poids total de la composition.

11. Procédé de traitement cosmétique des cheveux et/ou du cuir chevelu, **caractérisé par le fait qu'**il consiste à appliquer sur les cheveux et/ou le cuir chevelu, une composition cosmétique telle que définie dans l'une quelconque des revendications 1 et 3 à 10, à laisser celle-ci en contact avec les cheveux et/ou le cuir chevelu, et éventuellement à rincer.

## Patentansprüche

1. Kosmetische Verwendung mindestens einer Verbindung der allgemeinen Formel (I) in einer wirksamen Menge in einer Zusammensetzung: 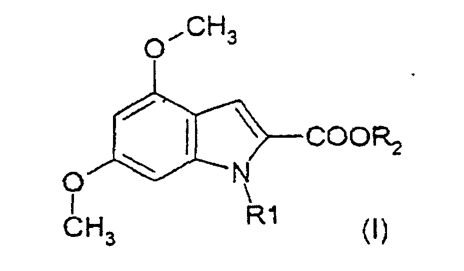 worin R₁ und R₂, die gleich oder verschieden sind, bedeuten:
ein Wasserstoffatom,
. eine C₁₋₆-Alkylgruppe, die gegebenenfalls mit -OH, -NHR₃, -SH, -COOH oder -COOR₃ substituiert ist, wobei R₃ eine geradkettige oder verzweigte C₁₋₄-Alkylgruppe bedeutet, eine C₇₋₁₂-Aralkylgruppe,
. eine Gruppe -CHR₄R₅, wobei R₄ und R₅, die gleich oder verschieden sind, ein Wasserstoffatom, eine gegebenenfalls substituierte Phenylgruppe oder einen 5- oder 6-gliedrigen Heterocyclus bedeuten;
der acylierten Formen dieser Verbindungen oder von physiologisch akzeptablen Salzen oder deren Gemischen in beliebigen Mengenanteilen;
zur Induzierung und/oder Stimulation des Haarwachstums und/oder zur Verlangsamung des Haarausfalls.

2. Verwendung mindestens einer Verbindung der allgemeinen Formel (I) nach Anspruch 1 in einer wirksamen Menge zur Herstellung einer pharmazeutischen Zusammensetzung zur Behandlung von Haarausfall.

3. Verwendung nach einem der Ansprüche 1 oder 2, **dadurch gekennzeichnet, dass** die Gruppe R₁ ein Wasserstoffatom; eine Methylgruppe oder eine Ethylgruppe bedeutet.

4. Verwendung nach dem vorhergehenden Anspruch, **dadurch gekennzeichnet, dass** R₁ ein Wasserstoffatom bedeutet.

5. Verwendung nach einem der vorhergehenden Ansprüche, **dadurch gekennzeichnet, dass** die Gruppe R₂ ein Wasserstoffatom oder eine Methylgruppe bedeutet.

6. Verwendung nach dem vorhergehenden Anspruch, **dadurch gekennzeichnet, dass** R₂ ein Wasserstoffatom bedeutet.

7. Verwendung nach einem der vorhergehenden Ansprüche, **dadurch gekennzeichnet, dass** die Verbindung ausgewählt ist unter:
4,6-Dimethoxy-indol-2-carbonsäure,
Methyl-4,6-dimethoxy-indol-2-carboxylat,
N-Methyl-4,6-dimethoxy-indol-2-carbonsäure,
N-Methyl-4,6-dimethoxy-indol-2-carbonsäuremethylester, und
N-Ethyl-4,6-dimethoxy-indol-2-carbonsäure.

8. Verwendung nach dem vorhergehenden Anspruch, **dadurch gekennzeichnet, dass** die Verbindung die 4,6-Dimethoxy-indol-2-carbonsäure ist.

9. Verwendung nach einem der vorhergehenden Ansprüche, **dadurch gekennzeichnet, dass** die Verbindung in einer Konzentration von 0,3 bis 30 Gew.-%, bezogen auf das Gesamtgewicht der Zusammensetzung, enthalten ist.

10. Verwendung nach einem der vorhergehenden Ansprüche, **dadurch gekennzeichnet, dass** die Verbindung in einer Konzentration von 0,5 bis 20 Gew.-%, bezogen auf das Gesamtgewicht der Zusammensetzung, enthalten ist.

11. Verfahren zur kosmetischen Behandlung der Haare und/ oder der Kopfhaut, das das darin besteht, eine kosmetische Zusammensetzung nach einem der Ansprüche 1 und 3 bis 10 auf die Haare und/oder die Kopfhaut aufzutragen, diese in Kontakt mit den Haaren und/ oder der Kopfhaut zu belassen und gegebenenfalls zu spülen.

## Claims

1. Cosmetic use, in a composition, of an effective amount of at least one compound corresponding to the general formula (I): 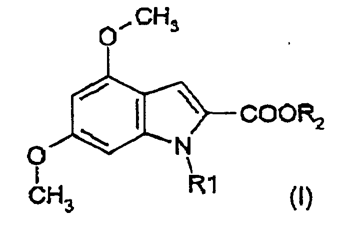 in which
R₁ and R₂, which may be identical or different, represent:
a hydrogen atom,
or a C₁-C₆ alkyl radical, optionally substituted with an -OH, -NHR₃, -SH, -COOH or -COOR₃ radical, in which R₃ represents a linear or branched C₁-C₄ alkyl radical,
or a C₇-C₁₂ aralkyl radical,
or a radical -CHR₄R₅, in which R₄ and R₅, which may be identical or different, represent a hydrogen atom or an optionally substituted phenyl radical or alternatively a 5- or 6-membered heterocycle;
the acylated forms thereof or the physiologically acceptable salts thereof, taken alone or as a mixture in any proportions;
for inducing and/or stimulating hair growth and/or preventing hair loss.

2. Use of an effective amount of at least one compound corresponding to the general formula (I) as defined in Claim 1 for the preparation of a pharmaceutical composition for treating hair loss.

3. Use according to either of Claims 1 and 2, **characterized in that** R₁ is a hydrogen atom or a methyl or even ethyl radical.

4. Use according to the preceding claim, **characterized in that** R₁ is a hydrogen atom.

5. Use according to any one of the preceding claims, **characterized in that** R₂ is a hydrogen atom or a methyl radical.

6. Use according to the preceding claim, **characterized in that** R₂ is a hydrogen atom.

7. Use according to any one of the preceding claims, **characterized in that** the compound is chosen from
4,6-dimethoxyindole-2-carboxylic acid
methyl 4,6-dimethoxyindole-2-carboxylate
N-methyl-4,6-dimethoxyindole-2-carboxylic acid
methyl N-methyl-4,6-dimethoxyindole-2-carboxylate
N-ethyl-4,6-dimethoxyindole-2-carboxylic acid.

8. Use according to the preceding claim, **characterized in that** the compound is 4,6-dimethoxyindole-2-carboxylic acid.

9. Use according to any one of the preceding claims, **characterized in that** the compound present is used at a concentration of between 0.3% and 30% by weight relative to the total weight of the composition.

10. Use according to any one of the preceding claims, **characterized in that** the compound present is used at a concentration of between 0.5% and 20% by weight relative to the total weight of the composition.

11. Cosmetic process for treating the hair and/or the scalp, **characterized in that** it consists in applying to the hair and/or the scalp a cosmetic composition as defined in any one of Claims 1 and 3 to 10, in leaving this composition in contact with the hair and/or the scalp, and in optionally rinsing it out.
